# EUROPEAN PATENT APPLICATION

(11) **EP 2 559 435 A1**
(43) Date of publication of application: **20.02.2013**
(21) Application number: 11006828.5
(22) Date of filing: 19.08.2011
(51) Int. Cl.: A61K 31/381, A61K 9/70, A61P 9/10, A61P 25/28

(54) **Rotigotine in the treatment of hemispatial neglect, stroke and deficits following stroke**

(71) Applicant: UCB Pharma GmbH, 40789 Monheim (DE)
(72) Inventor: Husain, Masud, London WC1N 3AR (GB)
(74) Representative: Krohn, Sabine Martha Erna

(57) **Abstract**

The present invention relates to the use of rotigotine for the effective treatment of a condition or disorder selected from neglect, stroke, conditions associated with stroke and/or following stroke. The invention also relates to a rotigotine-containing pharmaceutical composition, like a transepicutaneous. pharmaceutical composition especially in the form of a Transdermal Therapeutic System (TTS).

## Description

The present invention relates to the use of rotigotine for the effective treatment of a condition or disorder selected from neglect, stroke, conditions associated with stroke and/or following stroke. The invention also relates to a rotigotine-containing pharmaceutical composition, like a transepicutaneous pharmaceutical composition especially in the form of a Transdermal Therapeutic System (TTS).

### BACKGROUND OF INVENTION

Acute stroke is one of the leading causes of death in the elderly population of the developed world. Moreover, individuals surviving the acute phase of a stroke often suffer from severe disabilities and handicaps thereafter. The number of years with a diminished quality of life caused by the sequelae of stroke is larger than that of any other disease in this age group except for dementia. Thus stroke is a heavy burden for the individual, their relatives, and society.

According to the US National Institute of Neurological Disorders and Stroke (NINDS) stroke occurs when the blood supply to part of the brain is suddenly interrupted or when a blood vessel in the brain bursts, spilling blood into the spaces surrounding brain cells. Brain cells die when they no longer receive oxygen and nutrients from the blood or there is sudden bleeding into or around the brain. The symptoms of a stroke include sudden numbness or weakness, especially on one side of the body; sudden confusion or trouble speaking or understanding speech; sudden trouble seeing in one or both eyes; sudden trouble with walking, dizziness, or loss of balance or coordination; or sudden severe headache with no known cause. There are two forms of stroke: ischemic - blockage of a blood vessel supplying the brain, and hemorrhagic - bleeding into or around the brain.

As a result, the affected area of the brain is unable to function, which might result in an inability to move one or more limbs on one side of the body, inability to understand or formulate speech, or an inability to see one side of the visual field.

The consequences of stroke are often profound, with many patients suffering from major cognitive and motor deficits. Together, these problems contribute to stroke being a huge economic burden for healthcare systems internationally. In the UK, more than a quarter of the population over the age of 45 will suffer a stroke, while in the US, 700,000 individuals have a stroke annually, with prevalence data demonstrating that there are over 5 million survivors of stroke in that country alone. Cognitive impairment is present in more than 30% of such long□term survivors of stroke but may affect more than 70% of individuals acutely after stroke 4. Motor deficits are present in more than 80% of stroke survivors.

Hemispatial neglect (also known as spatial neglect, unilateral neglect or simply 'neglect') represents one of the major cognitive disorders following stroke. It is far more prominent and long-lasting in individuals with right hemisphere damage compared to those with left-hemisphere stroke. Neglect may affect more than 60% of right-hemisphere patients in the acute phase of stroke. Such patients often fail to be aware of objects - even people - towards their left. Most such patients with neglect also have significant weakness and impaired motor control of the left arm and leg, suffering a combination of cognitive and motor deficits which serves to compound their functional disability.

Neglect is a syndrome consisting of several component deficits, with different patients suffering different combinations of cognitive impairment. For example, difficulties In disengaging or directing spatial attention, initiating or executing movements, sustaining attention over time and representing space to the left have all been reported In Individuals with the syndrome. A potentially important component demonstrated in recent studies is a deficit in remembering spatial locations over brief periods of time - spatial working memory - which can interact with deficits in sustained attention to exacerbate neglect.

Currently, there are no reliable drug treatments for neglect, other cognitive impairments or motor deficits following stroke.

Recent studies have suggested that specialized care (including rehabilitation therapy) in stroke units improves outcomes after stroke (Langhome et al. Lancet 2011 377(9778):1693-702). Stroke rehabilitation is the process by which a stroke survivor works with a team of health care providers with the aim of regaining as much of the function lost after a stroke as possible. Some of the different types of medical professionals who participate in the care of stroke patients during the rehabilitation process include Physical Medicine and Rehabilitation Physicians (Physiatrists). Physical Therapists, Speech Therapists, Occupational Therapists. A comprehensive rehabilitation program is time consuming and cost-intensive therapy. It may last many weeks or even months.

There is an urgent need for pharmaceutical drugs and formulations providing time- and cost-efficient treatment of stroke and/or conditions associated with stroke or following stroke, thereby improving the patient's life and avoiding long term costs for other therapies and/or medical care.

The therapeutic goals in the treatment of acute stroke may be diverse. Therapeutic agents in the field of acute stroke can be expected to improve functional / global outcome (e.g. disability and handicap), i.e. increase patients' independence in basic and instrumental activities of daily living and/or improve neurological deficits.

There have previously been a few attempts to test modulation of dopaminergic activity as a therapeutic option in hemispatial neglect, but the largest trial tested only four patients. Despite some Initial promising results from an open-label study using bromocriptine, a dopamine D2 receptor agonist, in two patients (Fleet WS et al.,Neurology. 1987;37(11):1765), a further small open-label trial and a case report revealed worsening of neglect with the drug (Grujic Z. et al., Neurology. 1998;51(5):1395, Barrett AM et al. Archives of Physical Medicine and Rehabilitation. 1999 May;80(5):600-603). Apomorphine, which has both D1 and D2 dopmanine receptor activity, induced a transient improvement in three out of four neglect patients tested (Geminiani G. et al., Journal of Neurology, Neurosurgery & Psychiatry. 1998;65(3):344). Finally, a small-scale trial of amantadine, another drug which has some dopamine agonist activity, In four neglect patients did not demonstrate any beneficial effect of the drug (Buxbaum LJ et al.. American Journal of Physical Medicine & Rehabilitation. 2007 Jul;86(7):527-537. ).

Due to these discouraging results in the prior art, there remains the need for a drug suitable to effectively treat and or improve conditions like neglect, stroke, conditions associated with stroke and/or following stroke.

It is therefore an objective of the Invention to provide a drug and a pharmaceutical formulation that treats stroke or enhances recovery from stroke.

Another objective of the invention is to provide a drug and a pharmaceutical formulation that treats or improves neglect and its cognitive components, including spatial working memory, selective and sustained attention.

Another objective of the invention is to provide a drug and a pharmaceutical formulation that treats or improves motor function.

Surprisingly and unexpectedly, it has been found that rotigotine and the prodrugs and pharmacologically acceptable salts thereof can be used in the treatment of neglect, stroke, conditions associated with stroke and/or following stroke.

A surprising discovery has shown that the monotherapeutic administration of a rotigotine-containing transepicutaneous composition especially when in the form of a pharmaceutical transdermal therapeutic system (TTS) composition leads to the suppression and reduction of the stroke symptoms, rehabilitation of stroke patients.

### SUMMARY OF THE INVENTION

The present invention relates to the use of rotigotine, one of its pharmaceutically acceptable salts and/or a prodrug or metabolite of rotigotine for the treatment of neglect, stroke, conditions associated with stroke and/or following stroke while avoiding the known drawbacks of traditional current-art therapies and to a pharmaceutical formulation, in particular a pharmaceutical formulation for transdermal administration.

In one embodiment rotigotine [(-)-5,6,7,8-Tetrahydro-6-[propyl[2-(2-thienyl)ethyl]amino]-1-naphtol] and/or a prodrug thereof and/or physiologically acceptable salts thereof is use in the treatment of a condition or disorder selected from neglect, stroke, conditions associated with stroke and/or following stroke.

In another embodiment a pharmaceutically composition comprising rotigotine [(-)-5,6,7,8-Tetrahydro-6-(propyl[2-(2-thienyl)ethyl]amino]-1-naphtol] and/or a prodrug thereof and/or physiologically acceptable salts thereof for use in the treatment a condition or disorder selected from neglect, stroke, conditions associated with stroke and/or following stroke is provided.

In one embodiment of the invention the use relates to the treatment of a condition or disorder which is selected from cognitive and/or motor deficits.

In another embodiment the condition or disorder is selected from spatial neglect, hemispatial neglect, unilateral neglect, left sided neglect, right sided neglect, visual deficits, motor deficits, selective attention deficits, sustained attention deficits.

In still another embodiment the stroke is an ischaemic stroke.

In a further embodiment the stroke is a haemorrhagic stroke.

In one embodiment the condition or disorder is motor deficits following stroke.

In one embodiment the condition or disorder is a cognitive deficit.

In one embodiment the visual search and/or visual spatial working memory and/or motor function and/or selective attention and/or sustained attention on the affected side is improved.

In another embodiment an improvement in spatial bias on a visual search task, vis a vis placebo treatment, of at least 8% is effected.

In another embodiment an improvement in finding visual targets to the left on a visual search task, vis a vis placebo treatment, of at least 12% is effected.

In another embodiment an improvement in performance on a spatial working memory task, vis a vis placebo treatment, is effected at least in some patients.

In another embodiment an improvement in performance on motor function on the affected side, vis a vis placebo treatment, is effected at least in some patients.

In one embodiment selective attention in neglect is modulated, especially in patients with extensive damage in the prefrontal cortex.

In a further embodiment the patient group treated is characterized by extensive damage in the prefrontal cortex.

In one embodiment spatial bias is treated and/or improved in the extensive prefrontal patient group.

In another embodiment the sustained attention deficit in the extensive prefrontal patient subgroup is treated and/or improved.

In another embodiment the patient group treated is characterized by minimal involvement of the prefrontal cortex in the stroke lesion.

In a further embodiment neglect is treated and/or improved in a patient subgroup with minimal prefrontal involvement.

In one embodiment the medication is suitable for the application of 0.5 to 10 mg of the rotigotine per day.

In another embodiment the treatment is a transepicutaneous treatment.

Another embodiment comprises a transdermal therapeutic system comprising a backing that is inert relative to the components of the matrix, a self-adhesive matrix layer containing (-)-5, 6, 7, 8-tetrahydro-6-[propyl [2-(2-thienyl)ethyl]amino]-1-naphtol, and a protective film that is to be removed prior to use, characterized in that the matrix
a) includes as a substrate a non-aqueous, acrylate- or silicon-based polymer adhesive,
b) has a solubility of >=5% (g/g) for its free base (-)-5, 6, 7, 8-tetrahydro-6-[propyl [2-(2-thienyl)Ethyl]amino]-1-naphtol, and
c) contains the free base (-)-5, 6, 7, 8-tetrahydro-6-[propyl [2-(2-thienyl)ethyl]amino]-1-naphtol in an effective quantity.

### BRIEF DESCRIPTION OF THE DRAWINGS

- Fig. 1: shows the randomisation of treatment allocation and permutation tests
- Fig. 2: shows the assessment of spatial neglect -Mesulam cancellation task
- Fig. 3: shows the assessment of spatial neglect - Touchscreen cancellation task
- Fig. 4: shows the difference in performance on Mesulam visual search task on and off Rotigotine for all patients
- Fig.5: shows the effect size of rotigotine treatment in the Mesulam task
- Fig.6: shows the difference in Mesulam visual search task performance on and off rotigotine in the two subgroups defined according to prefrontal damage
- Fig.7: shows selective and sustained attention task and result for left targets

### DETAILED DISCRIPTION

A stroke, previously known medically as a cerebrovascular accident (CVA), is the rapidly developing loss of brain function(s) due to disturbance in the blood supply to the brain.

Conditions associated with stroke comprise spatial neglect, hemispatial neglect, unilateral neglect, left sided neglect, right sided neglect, cognitive deficits following stroke, visual deficits following stroke, selective and sustained attention deficits following stroke, motor deficits following stroke.

Rotigotine is the International Non-Proprietary Name (INN) of the compound (-)-5,6,7,8-tetrahydro-6- [propyl- [2- (2thienyl)ethyl]-amino]-1-naphthalenol, having the structure shown below

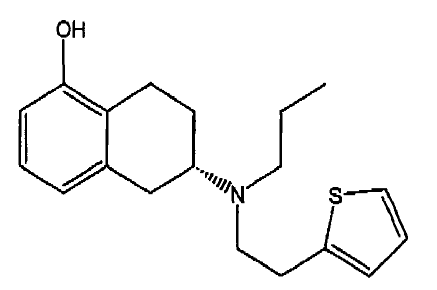

Two crystalline forms of rotigotine are presently known: polymorphic form I and polymorphic form II (WO 2009/068520). They can be differentiated by their respective physicochemical parameters, i.e. differing powder X-ray diffraction spectra, Raman spectra and melting points. At room temperature, the crystalline polymorphic form II is more stable than form I, which in turn is more stable than the amorphous form of rotigotine.

Rotigotine is a non-ergolinic D1/D2/D3 dopamine agonist, in particular D2 receptor agonist that resembles dopamine structurally and has a similar receptor profile but a higher receptor affinity.

Rotigotine is disclosed as active agent for treating patients suffering from Parkinson's disease (WO 2002/089777), Parkinson's plus syndrome (WO 2005/092331), depression (WO 20051009424) and the restless-legs syndrome (WO 2003/092677) as well as for the treatment or prevention of dopaminergic neurone loss (WO 2005/063237).

Known pharmaceutical compositions containing Rotigotine comprise a transdermal therapeutic system (TTS) (described in WO99/49852 or WO2011/076879), a depot form (described in WO 02/15903), an iontophoretic device (described in WO 2004/050083) and an intranasal formulation (described in WO 2005/063236).

Rotigotine has been tested in the form of its free base or as rotigotine hydrochloride.

In one embodiment, the method comprises administering rotigotine free base. In another preferred embodiment, the method comprises administering an acid addition salt of rotigotine, even more preferably rotigotine HCl. Other pharmaceutically acceptable acid addition salts include the oxolinate, tartrate, citrate, phosphate, sulfate and methanesulfonate salts.

In a further embodiment, the compound administered is a prodrug of rotigotine. A prodrug is an agent that generally has weak or no pharmaceutical activity itself but is converted into a pharmaceutically active compound in vivo. Prodrugs are often useful because, in some situations, they may be easier to administer than the corresponding active compound. A prodrug may, for instance, be bioavailable by oral administration where the active compound is not. A prodrug may be simpler to formulate, for example through improved solubility in a pharmaceutical composition, than the active compound.

As a nonlimiting example, prodrugs useful herein can be derivatives of rotigotine at the phenolic hydroxy group thereof, for example, esters (e.g., aryl carbonyl esters, alkyl carbonyl esters or cycloalkyl carbonyl esters, in particular alkyl carbonyl esters and cycloalkyl carbonyl esters each with up to 6 carbon atoms, carbonates, carbamates, acetals, ketals, acyloxyalkyl ethers, phosphates, phosphonates, sulfates, sulfonates, thiocarbonyl esters, oxythiocarbonyl esters, thiocarbamates, ethers and silylethers).

Alkyl carbonyl esters comprise compounds in which the oxygen atom of rotigotine is bonded to a -C(O)-alkyl group. An alkyl carbonyl ester can be formed by esterification of the phenolic hydroxy group of rotigotine with an alkanoic acid, e.g., with acetic acid, propionic acid, butyric acid, isobutyric acid or valeric acid.

Cycloalkyl carbonyl esters comprise compounds in which the oxygen atom of rotigotine is bonded to a -C(O)-cycloalkyl group.

Aryl carbonyl esters comprise compounds in which the oxygen atom of rotigotine is bonded to a -C(O)-aryl group.

Carbonates comprise compounds in which the oxygen atom of rotigotine is bonded to a - C(O)-O-R group, where R is as defined below.

Carbamates comprise compounds in which the oxygen atom of rotigotine is bonded to a - C(O)-NRR¹, -C(O)-NH-R¹ or -C(O)-NH₂ group, where R and R¹ are as defined below.

Acetals comprise compounds in which the oxygen atom of rotigotine is bonded to a - CH(OR)R¹ group, where R and R¹ are as defined below.

Ketals comprise compounds in which the oxygen atom of rotigotine is bonded to a - C(OR)R¹R² group, where R, R¹ and R² are as defined below.

Acyloxyalkyl ethers comprise compounds in which the oxygen atom of rotigotine is bonded to a -CHR-O-C(O)-R¹ or -CH₂-O-C(O)-R¹ group, where R and R¹ are as defined below.

Phosphates comprise compounds in which the oxygen atom of rotigotine is bonded to a - P(O₂H)OR group, where R is as defined below.

Phosphonates comprise compounds in which the oxygen atom of rotigotine is bonded to a - P(O₂H)R group, where R is as defined below.

Sulfates comprise compounds in which the oxygen atom of rotigotine is bonded to a - S(O)₂OR group, where R is as defined below.

Sulfonates comprise compounds in which the oxygen atom of rotigotine is bonded to a - S(O)₂R group, where R is as defined below.

Thiocarbonyl esters comprise compounds in which the oxygen atom of rotigotine is bonded to a -C(=S)-R group, where R is as defined below.

Oxythiocarbonyl esters comprise compounds in which the oxygen atom of rotigotine is bonded to a -C(=S)-O-R group, where R is as defined below.

Thiocarbamates comprise compounds in which the oxygen atom of rotigotine is bonded to a -C(=S)-N-RR¹, -C(=S)-NH-R¹ or -C(=S)-NH₂ group, where R and R¹ are as defined below.

Ethers comprise compounds in which the oxygen atom of rotigotine is bonded to a -R group, where R is as defined below.

In the above examples of prodrugs, each of R, R¹ and R² is independently hydrogen, alkyl (e.g., C₁₋₆ alkyl), cycloalkyl (e.g., C₃₋₁₀ cycloalkyl) or aryl (e.g., phenyl). In some embodiments, each of R. R¹ and R² is independently C₁₋₆ alkyl, C₃₋₁₀ cycloalkyl or phenyl.

Alkyl groups can be branched or unbranched and typically have 1 to 10 carbon atoms, for example C₁₋₆ alkyl. Alkyl groups can be unsubstituted or substituted with one or more substituents, for example halogen substituents.

Cycloalkyl groups may have only ring-forming C atoms or may optionally bear further C atoms. Illustratively, cycloalkyl groups have 3-10, 4-8 or 4-6 C atoms.

Phenyl groups can optionally be substituted in one or more positions (e.g., with alkoxy, alkyl, halogen and/or nitro substituents).

Illustrative prodrugs of rotigotine are described, for example, in the publications individually cited below.
Den Daas et al. (1990) Naunvn Schiedebergs Arch. Pharmacol. 341 : 186-191,
Den Daas et al. (1991) J. Pharm. Pharmacol. 43: 11-16.

Rotigotine is the (S)-enantiomer of 5,6,7,8-tetrahydro-6-[propyl[2-(2-thienyl)ethyl] amino-1-naphtol. This means that the content of (R)-enantiomers in the pharmaceutical composition is low according to the invention. The (R)-enantiomer is preferably present with a content of < 10 mole %, particularly preferably with a content of < 2 mole % and quite particularly preferably with a molar content of < 1%, based on the total quantity of rotigotine, in the pharmaceutical composition.

Rotigotine polymorphic form I or polymorphic form II, may be employed as drug.

The suitability of a prodrug of rotigotine can, for example, be determined by incubating a particular prodrug candidate under defined conditions with an enzyme cocktail and a cell homogenizate or an enzyme-containing cell fraction, and measuring the active rotigotine. A suitable enzyme mixture is for example the S9 liver preparation distributed by Gentext of Wobum, MA. Other methods to test the suitability of a prodrug are known to those skilled in the art. For example, in vitro conversion of a prodrug into the active substance can be assayed in the following way. The microsome fraction containing essential metabolic enzymes is obtained from liver cell homogenizates from humans, monkeys, dogs, rats and/or mice by differential centrifugation; alternatively, it is also possible to obtain the cytoplasmic fraction. The subcellular fraction is suspended with a buffer in such a way that a solution with a defined protein content is obtained. After the addition of 1 µM of the prodrug to be tested, it is incubated at 37°C for 60 minutes. Then rotigotine is quantified by means of HPLC/UV or HPLC/MS and related to the quantity used. For more detailed analyses, concentration or time series are investigated.

In a further embodiment, the compound administered is a metabolite of rotigotine. An example of such a metabolite of rotigotine is (S)-2-N-propylamino-5-hydroxytetralin, as disclosed for example in International Patent Publication No. WO 2005/058296.

Rotigotine and its prodrug can be present as free bases or in the form of physiologically acceptable salts, e.g. in the form of hydrochloride, in the medicament. "Physiologically acceptable salts" include non-toxic addition salts of rotigotine with organic or inorganic acids, e.g. HCl, HBr.

It has now been found that administering (-)-5, 6, 7, 8-tetrahydro-6-[propyl [2-(2-thienyl)ethyl]amino]-1-naphtol has a very beneficial effect on the syndromes known as neglect (hernispatial neglect, spatial neglect, unilateral neglect), stroke, conditions associated with stroke and/or following stroke.

Surprisingly, the compound to be used according to this invention already shows clear efficacy even in very low dosages and within a short time frame, making it suitable for use in the treatment of neglect, stroke, conditions associated with stroke and/or following stroke. Both in terms of its effective strength and its specificity this compound is superior to the substances that have so far been tested to be effective when the treatment of neglect, stroke, conditions associated with stroke and/or following stroke was indicated.

It lends itself particularly well to the treatment of neglect (hemispatial neglect, spatial neglect, unilateral neglect), stroke, conditions associated with stroke and/or following stroke.

The (-)-5,6,7,8-tetrahydro-6-[propyl [2-(2-thienyl)ethyl]amino]-1-naphtol compound is prepared in essentially conventional fashion. This compound can be prepared employing the method described in EP 0 168 505 B1. For the purpose of this invention, reference is made to that document in its entirety.

The advantage offered by the invention lies in the fact that the use of rotigotine as the active ingredient for treating neglect (hemispatial neglect, spatial neglect, unilateral neglect), stroke, conditions associated with stroke and/or following stroke allows even low dosages to improve the patient's condition without causing intolerable, undesirable effects (side effects). Suitable doses of rotigotine are between 0.05 and approximately 50 mg/day, whereby preferably daily doses of between 0.1 and 40 mg and in particular between 0.2 and 20 mg/day are administered. In one embodiment the active substance is applied in quantities of 0.5 to 10 mg per day, in another embodiment the active substance is applied in quantities of 1.0 to 10 mg per day, in still another embodiment the active substance is applied in quantities of 0.5 to 5 mg per day. In this regard, the dose can be increased gradually, which means that the treatment can optionally be commenced with low doses which are then increased to the maintenance dose.

It is evident to a person skilled in the art that the dosage interval may be varied according to the quantity applied, the method of application and the patient's daily requirement. For example, a formulation for transdermal application is conceivable, for example for once-a-day, three-day or seven-day administration while a subcutaneous or intramuscular depot can permit injections in a one, two or four-weekly cycle, for example.

In the pharmaceutical form, in addition to rotigotine, other active substances can be present which for example are used to treat high blood pressure, diabetes, clotting or thrombotic tendencies or high cholesterol, to prevent stroke. Examples of this include substances with an antihypertensive, antiplatelet, anticoagulant and/or thrombolytic, hypoglycaemic or cholesterol-lowering effect.

Antiplatelet drugs include for example aspirin, aspirin combined with dipyridamole, clopidogrel, coumadin is an example of ananticoagulant drug. Cholesterol lowering drugs include statins, while antihypertensive drugs include angiotensin II receptor blockers (ARBs), Angiotensin-converting enzyme (ACE) inhibitors, Beta-blockers, Calcium channel blockers, Diuretics.

In a combination preparation, the release of the active substances used in each case can take place to a large extent simultaneously or even sequentially. Sequential administration can, for example, be achieved by a pharmaceutical form, e.g. an oral tablet, having two different layers with different release profiles for the different pharmaceutically active components. A combination preparation according to the invention comprising a rotigotine formulation can alternatively also take the form of a "kit of parts" in which the respective active substances to be administered are present in formulations which are separate from each other, which are then administered simultaneously or in a temporally graduated manner.

It is evident to a person skilled in the art that, in the context of the present invention, different pharmaceutical forms and application schedules are conceivable, all of which are objects of the invention.

There are various methods of application available for the administration of rotigotine and its prodrugs which a person skilled in the art can select and adjust according to the requirement, condition and age of the patient, the required dose and desired application interval. Rotigotine may be orally or parenterally administered.

In general, it is parenterally administered, e.g. in the form of a transdermal therapeutic system (TTS), by injection, such as in the form of a depot suspension, by an iontophoretic device or in the form of an intranasal formulation.

If, a medicinal product in the form of a subcutaneous or intramuscular depot form is desired, the rotigotine can, for example, be suspended as a salt crystal, e.g. as a crystalline hydrochloride, in a hydrophobic, anhydrous medium and injected, as described in WO 02/15903, or also administered in the form of microcapsules, microparticles or implants based on biodegradable polymers, such as is described, for example, in WO 02/38646.

Other conceivable forms for the administration of rotigotine and its prodrug are transmucosal formulations, e.g. sublingual or nasal sprays, rectal formulations or aerosols for pulmonary administration.

In one embodiment rotigotine is transdermally administered. The pharmaceutical form can in principle be selected from, for example, ointment, paste, spray, film, plaster or an iontophoretic device.

The active substance is applied on the patient's skin in transepicutaneous form as an ointment, a gel or a cream, but preferably as a Transdermal Therapeutic System (TTS) in the form of a patch.

In one embodiment, the pharmaceutical formulation is a transdermal therapeutic system (TTS) comprising a backing layer that is inert with respect to the components of the matrix, a self-adhesive matrix layer containing (-)-5,6,7.8-tetrahydro-6-[propyl[2-(2-thienyl)ethyl]amino]-1-naphthol, and a protective film to be removed before use, characterised in that said matrix layer.

In another embodiment the TTS contains a backing that is inert relative to the matrix content, a self-adhesive matrix layer containing (-)-5, 6, 7, 8-tetrahydro-6-[propyl [2-(2-thienyl)ethyl]aminol-1-naphtol, and a protective film that must be peeled off before use, the system being characterized in that the matrix layer
a) includes a substrate layer of a non-aqueous acrylate- or silicone-based polymer adhesive,
b) features a solubility of >=5% (g/g) for the free base (-)-5,6,7,8-tetrahydro-6-[propyl [2-(2-thienyl)ethyl]amino]-1-naphtol, and
c) contains said free base (-)-5,6,7,8-tetrahydro-6-[propyl [2-(2-thienyl)ethyl]amino]-1-naphtol in an effective amount.

In an enhanced variation, the TTS matrix contains <0.5% (g/g) of inorganic silicate particles.

In a particularly desirable version the matrix of the Transdermal Therapeutic System contains <0.05% (g/g) of inorganic silicate particles.

In one embodiment of the invention, the Transdermal System includes an acrylate-based polymer adhesive that contains at least two of the following monomers: Acrylic acid, acrylamide, hexyl acrylate, 2-ethylhexyl acrylate, hydroxyethyl acrylate, octyl acrylate, butyl acrylate, methyl acrylate, glycidyl acrylate, methacrylic acid, methacrylamide, hexylmethyl acrylate, 2-ethylhexyl methacrylate, octyl methacrylate, methylmethacrylate, glycidyl methacrylate, vinyl acetate or vinyl pyrrolidone.

In an advantageously enhanced version of the invention the Transdermal System includes a silicone-based polymer adhesive with additives that improve the solubility of (-)-5,6,7,8-tetrahydro-6-[propyl [2-(2-thienyl)ethyl]amino]-1-naphtol, in the form of hydrophilic polymers or glycerin or glycerin derivatives.

In another implementation of the invention, the Transdermal System (-)-5,6,7,8-tetrahydro-6-[propyl [2-(2-thienyl)ethyl]amino]-1-naphtol includes a polymer adhesive at a concentration of 10 to 35% [g/g] if acrylate-based or of 5 to 40% [g/g] if silicone-based.

In another enhanced version of the invention, the Transdermal System contains substances that improve the permeation of (-)-5,6,7,8-tetrahydro-6-[propyl [2-(2-thienyl)ethyl]amino]-1-naphtol into the human skin.

According to the invention, the permeation-promoting substance added to the Transdermal System is selected from among the lipidols, fatty acids, fatty acid esters, fatty acid amides, glycerin or its derivatives, N-methylpyrrolidone, terpenes or terpene derivatives.

In one implementation of the invention, the permeation-promoting substance in the Transdermal Therapeutic System is oleic acid or oleyl alcohol.

According to the invention, a desirable hydrophilic polymer in the Transdermal Therapeutic System is polyvinyl pyrrolidone, a copolymer of vinyl pyrrolidone and vinyl acetate, polyethylene glycol, polypropylene glycol or a copolymer of ethylene and vinyl acetate.

In another implementation, the hydrophilic polymer contained in the Transdermal Therapeutic System is in the form of soluble polyvinyl pyrrolidone at a concentration of 1.5 to 5% (g/g) within the drug-containing matrix layer.

According to the invention, the matrix of the Transdermal System can additionally contain inert, cohesion-improving fillers.

One method for producing a Transdermal Therapeutic System according to the invention includes the following procedural steps:
i) Mixing of a suspension of (-)-5, 6, 7, 8-tetrahydro-6-[propyl [2-(2-thienyl)ethyl]amino]-1-naphtol hydrochloride In ethanol with an alkaline compound in ethanol for converting the hydrochloride into its free base;
ii) Filtering of the resulting suspension if and as necessary;
iii) Addition of polyvinyl pyrrolidone and an adhesive solution; and
iv) Drying of the product;
v) According to one implementation of the invention, the alkaline compound used in this process is sodium hydroxide or potassium hydrate.

In another implementation of the invention, the alkaline compound employed is sodium- or potassium metasillcate or trisllicate.
Finally, as part of the process prior to the drying of the product, the mixture can be coated onto an inert backing or protective film in such fashion as to produce a uniform layer.
As a result, the product obtained by the method described offers any one or a combination of the aforementioned features.
The Transdermal Therapeutic System can be produced as described in detail in the implementation examples of EP 1 033 978 B1.

A pharmaceutical product per this invention includes a reinforcing layer that is inert relative to the constituent components of the matrix, a self-adhesive matrix layer that contains an effective amount of rotigotine or rotigotine hydrochloride, and a protective film that must be removed before the product is used.

The following numbered paragraphs are of interest to or illustrative of the invention:
1. TTS comprising a backing layer that is inert with respect to the components of the matrix, a self adhesive matrix layer containing (-)-5,6,7,8-tetrahydro-6-[propyl[2-(2-thienyl)ethyl]amino]-1-naphthol, and a protective film to be removed before use, characterised in that said matrix layer.
2. TTS according to paragraph 1 wherein the TTS comprises a backing layer that is inert with respect to the components of the matrix, a self-adhesive matrix layer containing (-)-5,6,7,8-tetrahydro-6-[propyl[2-(2-thienyl(ethyl]amino]-1-naphthol, and a protective film to be removed before use, characterised in that said matrix layer
   a) contains, as the base, a non-aqueous acrylate or silicone-based polymer adhesive,
   b) has a solubility of ≥ 5% (g/g) for the free base (-)-5,6,7.8-tetrahydro-6-[propyl[2-(2-thienyl)ethyl]amino]-1-naphthol, and
   c) contains the free base (-)-5,6,7,8-tetrahydro-6-[propyl[2-(2-thienyl)ethyl]amino]-1-naphthol in an effective amount.
3. TTS according to any of the paragraphs 1 or 2 comprising at least one amine-compatible silicone pressure sensitive adhesive.
4. TTS according to any of the paragraphs 1 to 3 further comprising an additive.
5. TTS according to any of the paragraphs 1 to 4 wherein the additive is polyvinylpyrrolidone (PVP).
6. TTS according to any of the paragraphs 1 to 5 wherein the weight ratio of rotigotine to polyvinylpyrrolidone is in a range from about 9:3.5 to about 9:6.
7. TTS according to any of the paragraphs 1 to 6 wherein the weight ratio of rotigotine to polyvinylpyrrolidone is in a range from about 9:4.
8. TTS according to any of the paragraphs 1 to 7 wherein rotigotine and polyvinylpyrrolidone are contained in a multitude of microreservoirs.
9. Use of a TTS according to any of the paragraphs 1 to 9 for the treatment of neglect, stroke, conditions associated with stroke and/or following stroke.

The following non-limiting examples illustrate the invention:

### EXAMPLE

### TTS Production

5 g Sodium metabisulfite were dispensed in 45 g water while stirring until a clear 10% w/w/ aqueous solution is obtained.
Polyvinyl pyrrolidone (Kollidon 90 F) (5 g) was added to 14.8 g anhydrous ethanol while stirring and leave the solution for swelling. After swelling the mixture is stirred until the polyvinyl pyrrolidone is entirely dissolved. After the complete dissolution of the polyvinyl pyrrolidone, an aqueous 10% w/w sodium metabisulfite solution (0.022 g), 0.05 g ascorbyl palmitate and 0.124 g all-rac-α-tocopherol are added. The mixture is stirred until a clear ethanolic PVP solution is obtained.
An ethanolic PVP solution as described above (15.998 g) are dissolved in anhydrous ethanol (42.33 g) and the mixture is heated to 30 - 40° C while stirring. Then (-)-5,6,7,8-tetrahydm-6-[propyl-[2-(2-thienyl)ethyl]-amino]-1-naphthalenol (rotigotine, 18 g, polymorphic form I) was added to this solution. The mixture is stirred and heated to 50 - 60° C.
105.75 g of an amine resistant high tack silicone adhesive (BIO-PSA® Q7-4301 mfd. by Dow Coming) (70 wt-% solution in heptane), 105.72 g of an amine resistant medium tack silicone adhesive (BIO-PSA® Q7-4201 mfd. by Dow Coming) (70 wt-% solution in heptane), 63.54 g of the PVP-rotigotine-solution obtained above were mixed and all components were stirred until a homogenous dispersion was obtained.
The dispersion was coated onto a suitable fluoropolymer coated polyester release liner (e.g. SCOTCHPAK® 9744) with a suitable doctor knife and the solvents were continuously removed in a drying oven at temperatures up to 80°C for about 30 min to obtain a drug-containing adhesive matrix of 50 g/m² coating weight The dried matrix film was laminated with a polyester-type backing foil (e.g. Hostaphan MN 19). The individual patches were punched out of the complete laminate in the desired sizes (e.g. 10 cm², 20 cm², 30 cm²) and sealed into pouches under the flow of nitrogen.

### Clinical Trial

A commercially available rotigotine TTS has been tested in a double-blind, randomised, placebo controlled trial of the dopamine agonist rotigotine in 16 patients aged over 18 years with hernispatial neglect and unilateral weakness following right hemisphere stroke.

Patients were assessed with a battery of standardised neglect tests, as well as with tests of working memory, selective and sustained attention and motor function. A replicated ABA double-blind, placebo-controlled N-of-1 randomised design (Edgington E, Onghena P. Randomization Tests (Statistics: a Series of Textbooks and Monographs). 4th ed. Chapman & Hall/CRC: 2007), which allows to evaluate the effectiveness of an intervention in small sample sizes. Each patient's performance was measured in three phases, each consisting of several assessment sessions: before treatment (phase A1), while receiving transdermal rotigotine (phase B) and after discontinuation of the drug - post treatment phase (phase A2). The exact duration of each phase was randomised across patients. Performance on rotigotine was compared with the pre-treatment baseline and post-treatment follow-up phases.

The effects of the drug were evaluated for the whole group of 16 patients, and, separately for two subgroups with different extent of involvement of the prefrontal cortex in the stroke lesion. The subgroups are defined according to the extent of involvement of the prefrontal cortex in the stroke lesion: a minimal prefrontal involvement subgroup (0%-15% of the prefrontal cortex affected) and an extensive prefrontal subgroup (33%-55% of the prefrontal cortex affected).

All patients receive placebo and drug at different stages of the trial, with the exact time at which drug is started and the duration of treatment randomised across individuals.

The duration of each phase was randomised within limits, such that, in each patient, A1+B+A2 consisted of a total of 20 assessment sessions. However, the precise durations of A1, B and A2 varied across individuals, with both patients and investigators blind to the precise duration of each of these phases in any given patient.

Phase A1 started on session 1 and its duration was randomised (across individuals) to between 5 and 9 days. Observations during this phase established the baseline performance. Phase B, when rotigotine was administered, could commence on day 6 to day 10, and its duration was a minimum of 7 and a maximum of 11 sessions. During treatment phase, a rotigotine 9 mg patch (4mg/24hr transdermal absorption) was applied daily and worn for 24 hours a day. Finally, phase A2, when patients were assessed after the discontinuation of rotigotine, was randomised to begin between sessions 13 and 17, and it lasted for the remaining 4 to 8 sessions.

For the purpose of placebo control, all patients received a placebo patch in the period between sessions 6-16, on the days they were not receiving rotigotine. Placebo and rotigotine patches were visually identical. All investigators, clinical staff, patients and carers were masked to treatment assignment.

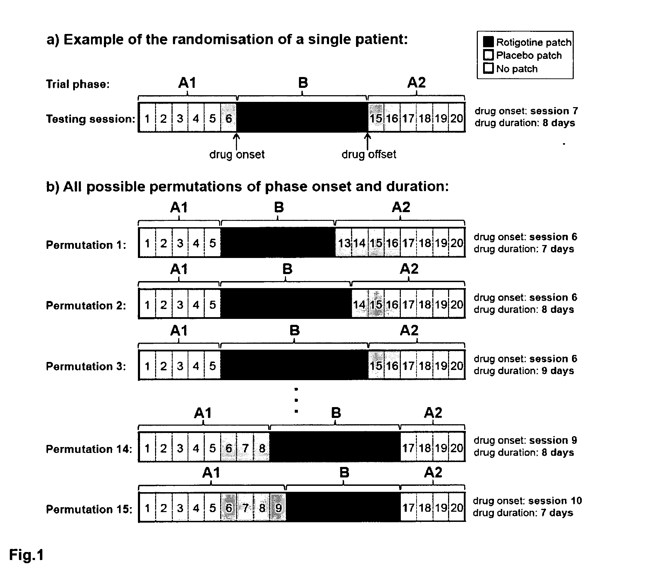

Each patient was randomly assigned a pattern of onset and duration of the treatment and baseline phases, within the duration limits described above. As an example, the randomisation profile of one of the participants is presented in figure 1 a. In this example, the patient had 6 baseline assessments (phase A1, sessions 1-6), followed by 8 days on rotigotine (sessions 7-14) and 6 follow-up assessments (phase A2, sessions 15-20) after discontinuation of the drug. In the figure the light grey shading shows the minimum number of sessions in phases A1 and A2, while dark grey shading denotes the treatment phase (B). Mid-grey depicts any additional sessions in phases A1 and A2 when the patient received placebo patches. Some other possible permutations of pre-treatment, treatment and post-treatment phases within the constraints of the design are shown in figure 1b. The actual difference in performance between treatment (B) and the off-treatment phases (A1 and A2) was ranked against the differences between phases produced by all other possible combinations of treatment allocation, given the limits in phase onset and duration. In total, there were 15 possible permutations.

Each patient participated in 20 consecutive assessment sessions. The first 17 sessions were performed daily. The final 3 follow-up assessments were conducted at weekly intervals. Each session consisted of tests of spatial neglect, spatial working memory and motor performance.

### Spatial Neglect And Component Deficits

### I. Mesulam cancellation (visual search) task of hernispatial neglect:

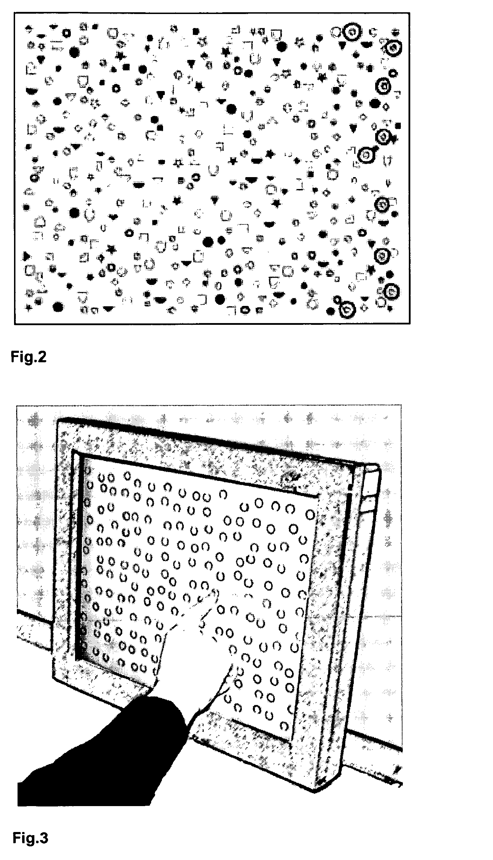

This visuospatial scanning test-further described in Mesulam, M.-M. Principles of Behavioural Neurology; tests of directed attention and memory (Davis, Philadelphia, 1985) - with a cluttered array of symbols is performed on A3 sheets. In this test visual target objects are identified among distracters; targets identified are marked by the participant (black circles in fig. 2). There was a 2 minutes time limit for this task. Left sided neglect patients with right hemisphere stroke demonstrate a spatial bias to the right, failing to find many targets to the left (before treatment).
In the group of all 16 patients, a significantly larger number of targets were identified on the left and in total when patients were on rotigotine.
Furthermore, on rotigotine, there was an improvement in the spatial bias of the responses, measured as the proportion of the targets identified on the left when compared to those identified on the right.
In the minimal prefrontal involvement subgroup, rotigotine had again a significant positive effect in the number of targets identified on the left. In the extensive prefrontal involvement subgroup, there was a significant improvement on rotigotine in the spatial bias.

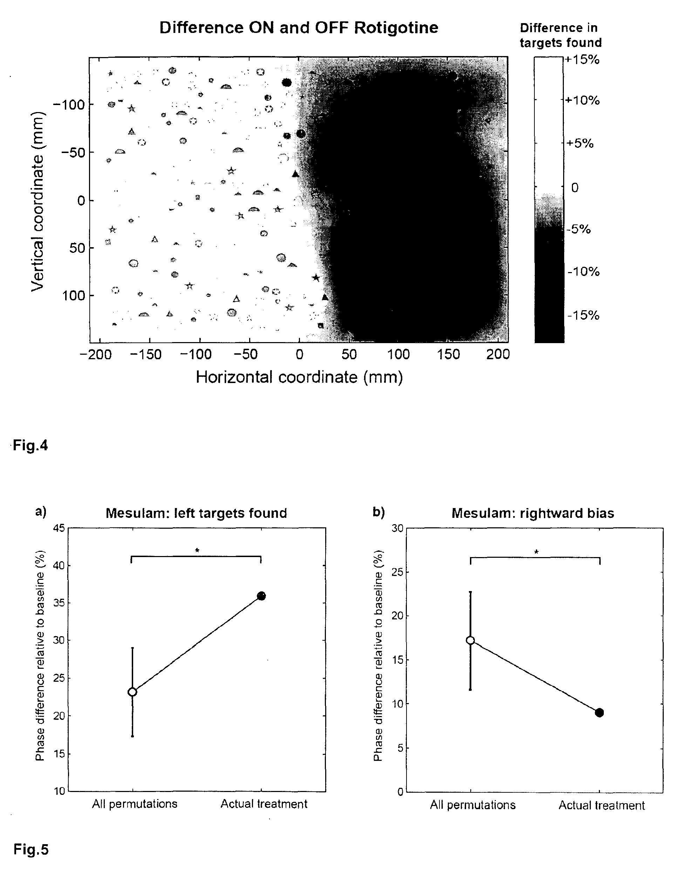

As shown in figure 4, for the entire group of 16 neglect patients, the number of targets found on the left side was significantly higher while on rotigotine than in the pre- and post-treatment phases averaged or in the post-treatment phase alone. The magnitude of this effect was considerable (figure 5a): the difference on- and off-treatment in the number of targets found on the left side relative to baseline was 12.8% higher in the actual treatment allocation than the mean difference between phases produced by all possible combinations of treatment onset and duration.

Spatial bias in visual search (ratio of difference in the number of targets found on either side to total number of targets found on the Mesulam test) also improved significantly on rotigotine when compared to the post-treatment phase or to both off-treatment phases (figure 4). The effect size was 8.1% less rightward bias relative to baseline in the actual treatment allocation, in comparison to all possible permutations (figure 5b).

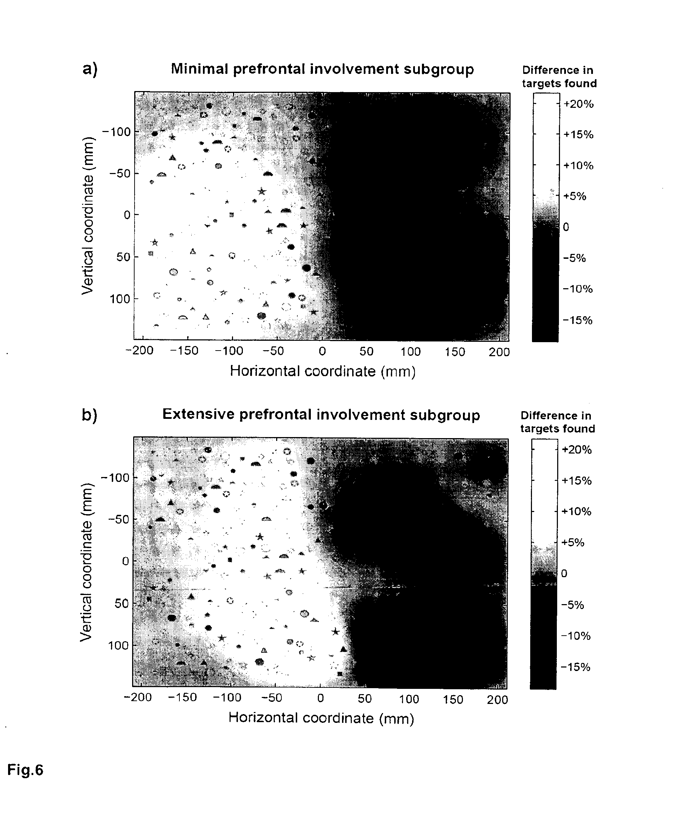

For both the minimal prefrontal involvement subgroup and extensive prefrontal involvement subgroup, there was a significant benefit of rotigotine (figure 6), but for different search parameters. The number of left targets found was significantly higher on rotigotine in the minimal prefrontal subgroup, but did not reach significance in the extensive prefrontal subgroup. Conversely, spatial bias improved significantly on rotigotine in the extensive prefrontal group, but not in the minimal prefrontal group.

Fig. 4 shows a heatmap of the difference in targets found on- and off-treatment for the entire patient group is overlaid on a Mesulam test sheet. Colour represents difference on- and off-treatment in number of targets found per session per patient at each target location. Treatment with rotigotine was associated with a significant increase in the number of targets identified on the left side. A decrease in the number of targets found during treatment in a smaller area on the right hand side was not statistically significant.

In fig. 5 the Y axes represent % difference between performance on treatment (phase B) and off-treatment (average of phases A1 and A2), relative to off-treatment baseline. The actual differences on- and off-treatment (in black) are compared to the average (±average SEM) of differences between phases B and the average of A1 and A2 produced by all possible combinations of the data (in grey).
a) The difference on- and off-treatment in the number of targets found on the left side relative to baseline was higher in the actual treatment allocation, when compared to all possible permutations.
b) There is less rightward lateralisation in the location of the targets found during treatment with rotigotine, in comparison to differences produced by all possible permutations of the data.

Fig. 6 shows the difference in Mesulam visual search task performance on and off rotigotine in the two subgroups defined according to prefrontal damage.
a) In the subgroup with minimal prefrontal involvement, the number of targets found on the left side increased significantly on treatment.
b) Patients with extensive prefrontal involvement showed a significant improvement In rightward spatial bias during treatment.

### II. Touchscreen cancellation task of hemispatial neglect:

In this version of search task-further described in Parton et al. Neuroreport. 2006 17(8):833-6 - targets are Identified as clicks or touches on a touchscreen; cancellations (found targets) are stored in the computer's memory, but no visible mark is left on the screen. Therefore this test also allows the possibility to measure whether subjects reclick on (revisit) previously cancelled targets, due to a deficit in memory of the targets' location (fig. 3).

In the group of all 16 patients, on average % more targets were identified while on rotigotine than before treatment onset.

### III. Vertical Corsi spatial working memory task:

In this test, the subject had to memorise the location of 1-5 objects presented vertically. This test was designed and validated for the assessment of spatial working memory (memory of objects' location) in patients with spatial neglect. It is further described in Malhotra, P. et al Spatial working memory capacity in unilateral neglect. Brain 128, 424-436 (2005).
In some patients rotigotine improved spatial working memory, as evidenced by an improvement in spatial span, measured by the vertical Corsi task.

### IV. Selective and sustained attention (vigilance) task:

The subject had to identify high- or low- sallence targets among non targets on either side. % correct and reaction times are measured, further described in Barcelo et al. Nature Neuroscience 2000 3:399-403.

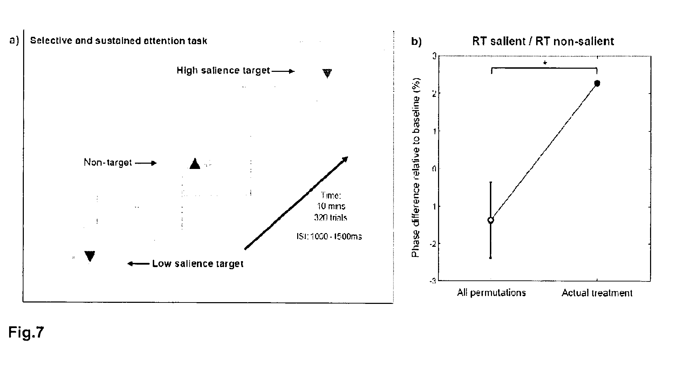

Participants detected targets (inverted triangles) among sequences of distractors (upright triangles) randomly presented to the ipsilesional and contralesional visual fields. Targets could be of the same colour as the distractors (dark grey - low visual salience) or of a different colour (light grey - high visual salience) (fig. 7a). Participants were asked to respond with a button press as soon as they saw a target of any type.

As a measure of selective attention, the ratio of the reaction time (RT) to high visual salience targets over the RT to low visual salience targets was used. Furthermore, using this task, sustained attention was quantified over time, by measuring the difference in RT and % correct responses between the first and the second half of each experimental session.

In fig. 7b (Effect of rotigotine treatment on selective attention for left sided targets) the Y axes represent % difference between performance on- (phase B) and pre-treatment (phase A1), relative to pre-treatment baseline. The actual differences on- and pre-treatment (in black) are compared to the average (±average SEM) of difference between phases B and A1 produced by all possible combinations of the data (in light grey). The difference on- and pre-treatment in the ratio of the reaction time (RT) to salient targets over non-salient targets on the left side relative to baseline was higher in the actual treatment allocation, when compared to all possible permutations.

For the entire group there was a significant increase in the ratio of RT to high salience vs low salience targets for left sided targets during treatment, in comparison to pre-treatment baseline (figure 7). This effect was only marginal when comparing treatment to post-treatment baseline, or to the average of both off-treatment phases. In the extensive prefrontal subgroup, rotigotine was associated with similar improvement for left targets, when compared to pre-treatment baseline or to the average of both treatment phases. In addition, it also had a significant beneficial effect overall for both left and right-sided targets. Conversely, in the minimal prefrontal subgroup, the effect of rotigotine had no significant effect (e.g., for left sided targets, comparison with off-treatment average), even though at baseline reaction times ratios were not significantly different between the two patient subgroups. Thus, rotigotine was associated with a modulation of selective attention in neglect, but this was far more prominent in patients with extensive damage in the prefrontal cortex.
Rotigotine improves selective voluntary attention.

### Motor Function On The Affected Side

### I. Motricity Index

This test used to assess the motor impairment in a patient who has had a o assess the motor impairment in a patient who has had a stroke, in particular of arm leg and trunk. It is described in more detail e.g. by Wade, D.T. Measurement in Neurological Rehabilitation, (Oxford University Press, Oxford, 1992)

Rotigotine improved motor function on the affected side in some patients, as evidenced by improvement in the Motricity Index of the affected arm, leg and side.

### Conclusions

In a group of patients having faced a stroke with hernispatial neglect and unilateral weakness following right hemisphere stroke, rotigotine significantly improves visual search (as evidenced by the significant improvement in Mesulam task) and selective attention (selective - sustained attention task) improved motor function on the affected side in some patients (as evidenced by improvement in the Motricity Index) as well as in some patients visual working memory (vertical Corsi task).

## Claims

1. Rotigotine [(-)-5,6,7,8-Tetrahydro-6-[propyl[2-(2-thienyl)ethyl]amino]-1-naphtol] and/or a prodrug thereof and/or physiologically acceptable salts thereof for use in the treatment of a condition or disorder selected from neglect, stroke, conditions associated with stroke and/or following stroke.

2. Pharmaceutical composition comprising rotigotine [(-)-5,6,7,8-Tetrahydro-6-[propyl[2-(2-thienyl)ethyl]amino]-1-naphtol] and/or a prodrug thereof and/or physiologically acceptable salts thereof for use in the treatment a condition or disorder selected from neglect, stroke, conditions associated with stroke and/or following stroke.

3. Use according to any of the preceding claims wherein the condition or disorder is selected from cognitive and/or motor deficits.

4. Use according to any of the preceding claims wherein the condition or disorder is selected from spatial neglect, hernispatial neglect, left sided neglect, right sided neglect, visual deficits, motor deficits, selective attention deficits, sustained attention deficits.

5. Use according to any of the preceding claims wherein the stroke is an ischaemic stroke.

6. Use according to any of the preceding claims wherein the stroke is a haemorrhagic stroke.

7. Use according to any of the preceding claims wherein the condition or disorder is motor deficits following stroke.

8. Use according to any of the preceding claims wherein the condition or disorder is a cognitive deficit.

9. Use according to any of the preceding claims wherein the visual search and/or visual spatial working memory and/or motor function and/or selective attention and/or sustained attention on the affected side is improved.

10. Use according to any of the preceding claims wherein an improvement in spatial bias on a visual search task, vis a vis placebo treatment, of at least 8% is effected.

11. Use according to any of the preceding claims wherein an improvement in number of targets found to the left on a visual search, vis a vis placebo, treatment of at least 12% is effected.

12. Use according to any of the preceding claims wherein an improvement in performance on a spatial working memory, vis a vis placebo treatment, is effected.

13. Use according to any of the preceding claims wherein an improvement in performance on motor function on the affected side, vis a vis placebo treatment, is effected.

14. Use according to any of the preceding claims, **characterized in that** said medication is suitable for the application of 0.5 to 10 mg of the rotigotine per day.
Use according to any of the preceding claims wherein the treatment is a transepicutaneous treatment.

15. Transdermal therapeutic system comprising a backing that is inert relative to the components of the matrix, a self-adhesive matrix layer containing (-)-5,6,7,8-tetrahydro-6-[propyl [2-(2-thienyl)ethyl]amino]-1-naphtol, and a protective film that is to be removed prior to use, **characterized in that** the matrix
a) includes as a substrate a non-aqueous, acrylate- or silicon-based polymer adhesive,
b) has a solubility of >=5% (g/g) for its free base (-)-5,6,7,8-tetrahydro-6-[propyl [2-(2-thienyl)ethyl]amino]-1-naphtol, and
c) contains the free base (-)-5,6,7,8-tetrahydro-6-[propyl [2-(2-thienyl)ethyl]amino]-1-naphtol in an effective quantity.
